**Europäisches Patentamt**

**European Patent Office**

**Office européen des brevets**

(11) Publication number : **0 218 309 B1**

# EUROPEAN PATENT SPECIFICATION

(45) Date of publication of patent specification :
**15.11.95 Bulletin 95/46**

(51) Int. Cl.⁶ : **G01N 33/53**, // G01N33/74

(21) Application number : **86300336.4**

(22) Date of filing : **17.01.86**

(54) Method for measuring free ligands in biological fluids.

Divisional application 95103930.4 filed on
17/01/86.
The file contains technical information
submitted after the application was filed and
not included in this specification

(30) Priority : **04.10.85 US 784857**

(43) Date of publication of application :
**15.04.87 Bulletin 87/16**

(45) Publication of the grant of the patent :
**15.11.95 Bulletin 95/46**

(84) Designated Contracting States :
**AT BE CH DE FR GB IT LI LU NL SE**

(56) References cited :
EP-A- 0 015 687
EP-A- 0 054 975
EP-A- 0 078 477
EP-A- 0 089 806

(56) References cited :
EP-A- 0 133 464
EP-A- 0 155 104
EP-A- 0 163 122
EP-A- 0 165 669
WO-A-85/00226
GB-A- 2 085 160
US-A- 3 928 553

(73) Proprietor : **DIAGNOSTIC PRODUCTS
CORPORATION**
**5700 West 96th Street**
**Los Angeles California 90045 (US)**

(72) Inventor : **Said El Shami, A.**
**29974 Rolling Ridge Drive**
**Agoura Hills, CA 91301 (US)**

(74) Representative : **Cresswell, Thomas Anthony
et al**
**J.A. KEMP & CO.**
**14 South Square**
**Gray's Inn**
**London WC1R 5LX (GB)**

## Description

BACKGROUND OF THE INVENTION

For several decades equilibrium dialysis techniques were the only available method for the measurement of free hormones in serum, and until recently were the only methods considered reliable. Equilibrium dialysis methods in this context suffer from several drawbacks including poor precision. tediousness and so on; but above all their results are highly dependent on the purity of the tracers used.

Ellis and Ekins, R. (Acta Endocr. (KbH.) Suppl. 177:106, 1973), disclosed a direct method for free hormone determinations in their paper "Direct Measurement By Radioimmunoassay of the Free Thyroid Hormone Concentration in Serum." This represented a major improvement over equilibrium dialysis methods because it allowed for the direct measurement by radioimmunoassay (RIA) of free ligand levels in serum dialysates, thus circumventing the problem of tracer purity. This method is now considered by many as the reference methodology for free hormone measurements. It is, however, still time consuming and operator-dependent, and it is unavailable to most small laboratories.

Indirect methods for the estimation of free hormone concentrations which were introduced shortly thereafter include the testosterone/steroid hormone binding globulin (SHBG) ratio, the thyroxine (T4)/thyroid binding globulin (TBG) ratio, the free T4 index (based on the product of triiodothyronine (T3) uptake and T4), and the free androgen index.

Ekins, R. (Free Thyroid Hormones; Proceedings of the International Symposium held in Venice, December 1978, Amsterdam: Excerpta Medica, 1979 72-92), introduced the concept of "direct dynamic methods" in which an anti-free ligand antibody is used in direct contact with the biological fluid during dialysis This constitutes the basis for so-called "immunoextraction" methods.

One such method is taught in U.S. Patent No. 4,046,870 in which a two-tube immunoassay method measures the rate of transfer of T4 from binding proteins to T4-specific antibody. This method suffered from several analytical and clinical shortcomings which made it virtually just another free T4 index assay.

A second method, introduced by Clinical Assays (Cambridge, MA 02139), was a true immunoextraction method. It used a single-tube, two-stage, sequential (back-titration) technique. In this method, a serum sample is incubated with immobilized antibody; then, following a wash step, unoccupied sites on the immobilized antibody are "back-titrated" using labeled ligand. In this approach, the serum is never in contact with the labeled ligand. Although theoretically sound, it suffers from poor sensitivity and precision, and both reactions require exact timing.

Single-step immunoextraction methods for the determination of free ligand concentrations in biological specimens were the obvious next step in the development of free ligand assay systems. These methods rely on chemical rather than physical separation of labeled ligand from endogenous binders. In order to achieve this objective, several approaches can be adopted, as detailed below.

The prior art discloses that by chemically altering the structure of a given ligand, its binding to endogenous binders is reduced or diminished. This has been amply demonstrated for steroid hormones. (See the discussion of free testosterone below.) In the case of thyroid hormones, Ross, J. E. and Tapley, D.F. (Effect of various analogues on the binding of labeled thyroxine to thyroxine-binding globulin and prealbumin, Endocrinology 79:493, 1966), have shown that the binding of TBG (thyroid binding globulin) to T4 is inhibited if a fairly bulky substitution is made at the 3' position of the T4 molecule. In addition, Schall, R. F., et al (An enzyme-labeled immunoassay for the measurement of unsaturated thyroid hormone binding capacity in serum and plasma, Clin. Chem. 25:1078 (abstract) 1979), and Kleinhammer, G., et al (Enzyme immunoassay for determination of thyroxine binding index, Clin. Chem. 24:1033, 1978), independently demonstrated that TBG fails to bind to conjugates formed by labeling T4 with horseradish peroxidase. This fact constitutes the basis for the single-step immunoextraction method described in U.S. Patent No. 4,410,633 to Corning Glass Works, for the measurement of free thyroxine and free 3,5,3'-triiodothyronine wherein horseradish peroxidase is chemically attached to T4 and T3 and later radiolabeled.

In addition, the prior art also discloses that T3 and T4 require the following molecular structure for maximal binding to endogenous binding proteins, viz. TBG, thyroid binding pre-albumin (TBPA), albumin, Snyder, S. M, et al (Binding of thyroid hormones and their analogues to thyroxine-globulin in human serum, J. Biol. Chem. 251:6489, 1976); Sterling, K., et al (Equilibrium dialysis studies of the binding of thyroxine by human serum albumin, J. Clin. Invest. 41:1021, 1962):

1. The L-alanine side chain configuration;
2. The presence of 4'-hydroxyl group (primarily for TBPA and albumin binding); and
3. The presence of two (halogen) substituents in the inner and outer rings (positions 3,5,3' and 5').

Several hundred T3 and T4 analogs have been synthesized and studied for their ability to bind to thyroid hormone binding proteins.

U.S. Patent No. 4,366,143 and its European counterpart, Patent No. 00 26 103, broadly describe the use of such analogs as tracers in a single immunoextraction using simultaneous rather than sequential titration of antibody for the measurement of free hormones. (For convenience, these patents will be collectively referred to hereinafter as the "Amersham" patent.)

An intact alanine side chain is required for optimal binding of T4 and T3 to TBG: the amino group on the alanine side chain is the essential constituent. Analogs described in the Amersham patent are T3 and T4 molecules modified at the alanine side chain. Although theoretically these analogs do not bind TBG to any significant extent, they undoubtedly bind albumin and TBPA significantly since the 4'-hydroxyl group on the T3 and the T4 molecules is left intact. It is well established that the binding of albumin and TBPA to the thyronines is quantitative, especially under physiological conditions, Sterling, K. (Molecular structure of thyroxine in relation to its binding by human serum albumin, J. Clin Invest. 43:1721, 1964), and Pages, et al (Binding of thyroxine and thyroxine analogs to human serum prealbumin, Biochem 12:2773, 1973).

The failure of the Amersham patent to recognize the importance of albumin and TBPA binding to the thyronines renders the patent's teachings inadequate for the true measurement of free T3 and free T4 in biological fluids. In fact the commercially available reagents based on the patent yield misleading and inaccurate free hormone results. This is particularly true in several pathological conditions characterized by significant alterations in the circulating albumin level.

Recent literature has shown that the albumin concentration correlates directly with free T4 concentrations generated by the Amersham assay system. In addition, it is well documented that Amersham's method consistently yields falsely decreased free T4 results in third-trimester pregnancies and in patients suffering from severe non-thyroidal illness, while yielding falsely elevated free T4 levels in cases of familial dysalbuminemic hyperthyroxinemia, a condition in which T4 is abnormally bound to circulating albumin.

During pregnancy, albumin circulates at lower than normal levels, especially during the third trimester. Since Amersham's labeled analog T4 tracer binds albumin and TBPA to a significant extent (greater than 99%), one would expect the Amersham assay system to yield lower than normal free T4 results during the third trimester: more analog tracer is available to bind T4 antibody, resulting in higher binding and lower apparent dose.

Non-esterified free fatty acids are capable of displacing labeled analog from albumin; moreover, they circulate at higher than normal concentrations during pregnancy. This could explain the lower than expected free T4 values encountered during pregnancy when assayed by the Amersham method; apparent free T4 levels would be significantly lower than expected if albumin binding to the labeled analog is substantial.

This situation is also well documented in cases of heparin therapy, where a significant elevation of non-esterified free fatty acids is present. Free T4 and free T3 levels when measured by Amersham's method on heparin-treated patients show lower than normal levels.

The same problem occurs for non-thyroidal illness, where free T3 and T4 values generated by the Amersham method have been shown to be significantly lower than for a euthyroid population, when compared to a direct equilibrium dialysis method.

EP-A-155 104 describes an assay for e.g. thyroxine (T4) in a biological fluid in which the T4 is partly bound to natural protein binders. Free T4 and a T4 derivative compete for reaction with a specific binder, and a differential blocking agent is used to reduce binding of the T4 derivative but not T4 to the natural protein binders.

The Amersham patent procedure has been found wanting by workers in the art as manifested by the observance of false and erroneous measurements of free ligand levels. Applicant has discovered that the problem stems from binding of the ligand analog tracer to certain endogenous proteins, e.g., albumin in biological fluids. I have discovered that this problem can be overcome by the use of specific chemical inhibitor reagents. This discovery represents a major advance in the art.

The invention provides a method for measuring the concentration of free thyroxine or triiodothyronine ligand in a biological fluid in the presence of bound ligand and endogenous binding proteins including albumin, without disturbing the equilibrium between free ligand and protein-bound ligand, which method comprises

(a) incubating a sample of the biological fluid with (i) a ligand analog tracer which, due to its chemical structure, does not bind to some of the endogenous binding proteins but does bind to at least one other endogenous binding protein including albumin, (ii) a concentration of a specific ligand binder having an affinity constant and selectivity for the free ligand such that the equilibrium between free ligand and protein-bound ligand is not disturbed and (iii) 25 mg/ml sodium salicylate and 0.15 mg/ml 2,4-dinitrophenol;

(b) separating the ligand analog tracer bound to the specific ligand binder from unbound tracer; and

(c) determining the concentration of free ligand in said biological fluid.

## Brief Description of The Drawings

Figures 1 to 16 show the results of Example 3 in which any effect of charcoal-absorbed human serum albumin (CAHSA) on the free T3 and T4 assay systems was determined.

Figures 17 to 20 show the results of Example 7 which demonstrates that the result generated by the free T4 assay are unaffected by pregnancy and non-thyroided illness.

The present invention addresses the deficiencies encountered in the Amersham patent and effectively corrects for the inconsistencies in free thyroid results generated by Amersham's analog method.

The present invention may use labeled analogs for T3 and T4 that are modified at the alanine side chain. Specifically, the $\alpha$-amino group may be modified to prevent their binding to TBG. Meanwhile, steps have been taken to prevent such labeled analogs from binding to albumin and TBPA. This is accomplished by using sodium salicylate and 2,4-dinitrophenol which are able to blind to unoccupied binding sites on the albumin and TBPA molecules, thus saturating these binding proteins and effectively eliminating their capacity to bind to thyronine analogs and to other endogenous substances such as non-esterified free fatty acids. Sodium salicylate and 2,4-dinitrophenol do not bind to TBG, and their concentration should be such as not to displace any bound hormone from albumin or TBPA.

The association constant for albumin and T4 is approximately 500,000. (This estimate is based on the assumption that the number of binding sites on the albumin molecule available for thyroxine is equal to 1, and that the apparent association constant in liters per mole - i.e. the equilibrium constant in the direction of complex formation - is $5 \times 10^5$.) Likewise, the association constant for albumin and T3 is approximately 24,600. It is well established that albumin has a higher affinity for free T3 and T4 and their analogs than for anionic dyes, but a much higher affinity for free fatty acids than T3 and T4 and their analogs.

Albumin has a relatively low association constant for single aromatic compounds; the highest association constants are for 2,4-dinitrophenol (11,000) and salicylate (2,800).

In order to maintain strict equilibrium conditions <u>in vitro</u> during the immunoextraction reaction one has to maintain strict physiological conditions; this entails the use of pH = 7.4. At that pH, thyronine molecules have three charged groups: the anionic carboxylate ion, the cationic $\alpha$-amino group and the anionic phenolate ion. (The latter is 82% ionized.) The presence of albumin or TBPA under these physiological conditions yields a highly charged albumin with a relatively large number of cationic amino groups. These cationic amino groups on the albumin molecule bind the anionic phenolate ion on the thyronine molecules. Such an interaction is the main cause of albumin binding to the labeled analog in both the Amersham patent method and the Corning patent method.

The present invention makes use of the fact that 2,4-dinitrophenol (DNP) and sodium salicylate with their relatively high association constants to albumin and TBPA will also be ionized and charged under these physiological conditions of pH, yielding charged anionic phenolate ion capable of interaction with the charges on the albumin and TBPA molecules. When 2,4-dinitrophenol and sodium salicylate are present in excess, the binding of labeled T3 and T4 analogs to albumin and TBPA is virtually eliminated. This method of blocking albumin and TBPA by appropriate concentrations of 2,4-dinitrophenol and/or sodium salicylate is an effective means for eliminating the erroneous assay results caused by albumin in free thyroid hormone immunoextraction analog methods.

In general, the specific ligand binder is one which couples or binds to the free ligand and it may be a specific antibody for the free ligand or other binding agent. Specific ligand binders are known and need not be further described.

The ligand analog tracer is labeled in some way so as to be detectable or observable. Radiolabels are well-known and applicable, as are the other labeling means previously employed in this art, including enzymes, fluorophors, chromophores and chemiluminescent groups integral with the ligand analog tracer molecule.

Antibodies to both L-thyroxine and 3,5,3'-triiodothyronine were produced in rabbits by well-established, conventional techniques using bovine serum albumin-T4 and -T3 as the immunogens.

Analogs of diiodothyronine (T2) and T3 were prepared by succinylating the $\alpha$-amino group on the analine side chain to produce N-L-diiodothyronine succinimide and N-L-triiodothyronine, respectively, which were then iodinated by conventional iodination procedures to produce, respectively, N-$^{125}$I-L-triiodothyronine succinimide and N-$^{125}$I-L-thyroxine succinimide. The tracers were then compounded in 0.01M HEPES (N-2-hydroxyethylpiperazine-N'-2-ethanesulfonic acid) buffer, pH 7.4 and 0.01% sodium azide. 0.1% charcoal-absorbed human serum albumin (CAHSA) free of any apparent T3 or T4, and blocking agents were added as described in specific examples below. Different amounts of T3 or T4 were added to human serum free of any apparent T3 and/or T4, calibrated in terms of direct equilibrium dialysis, and assigned values for each level.

T3 and T4 antibodies were immobilized on the inner walls of polypropylene 12X75mm tubes by passive adsorption as described in Catt, K., et al (Solid phase radioimmuoassay in antibody-coated tubes, Science 158:1570, 1967).

For the assay of free T4, 50 μl of calibrator or patient sample is pipetted into anti-T4 antibody-coated tubes, followed by 1.0 ml of the labeled T4 analog. The tubes are then incubated for 60 minutes at 37°C. After this incubation the tubes are decanted and the bound radioactivity is counted. Results are calculated from the calibration curve and expressed in ng/dl.

For free T3 assay, 100 μl of calibrator or patient sample is pipetted into anti-T3 antibody-coated tubes, followed by 1.0 ml of labeled T3 analog tracer. The tubes are incubated for three hours at 37°C, then decanted and radioactivity counted. The results are calculated as for free T4 and expressed in pg/ml.

Example 1:

The choice of antibodies for the free T3 and free T4 assay systems was determined by the fact that the free hormone is in physiological equilibrium with its transport proteins. This equilibrium should be maintained when an antibody directed against the hormone is added to the system. It is essential to select an antibody which is appropriate in terms of its affinity constant and its specificity for the free analyte. Such antibodies should also have slow reaction kinetics.

For free thyroxine (T4) an antibody with a working titer or dilution of 1:250,000 was selected (2.0 ng IgG/tube). In order to check the effect of tracer binding to the antibody in the presence and absence of albumin and albumin-blocking agents, antibody-coated tubes were prepared using titers of 1:250,000 (2.0 ng IgG/tube) and 1:25,000 (20.0 ng IgG/tube). Maximum bindings were determined following the free T4 protocol described above. The results are tabulated below in table 1.

Table 1. Free T4.

| Ab Titer: | % B/T | | |
|---|---|---|---|
| | 1:25,000 | 1:250 000 | |
| Tracer A | 60.8% | 63.3% | without CAHSA: no zero calibrator (system devoid of albumin) |
| Tracer B | 18.1% | 2.6% | without CAHSA: 1.0 mg albumin/tube contributed from zero calibrator (50 μl) |
| Tracer C | 15.0% | 1.4% | with 1 mg CAHSA/tube: no zero calibrator |
| Tracer D | 9.4% | 0.7% | with 1 mg CAHSA/tube + 50 μl zero calibrator |
| Tracer E | 39.1% | 23.2% | with 1 mg CAHSA/tube + 50 μl zero calibrator + 0.5 mg/ml Na salicylate |
| Tracer F | 53.5% | 49.2% | with 1 mg CAHSA/tube + 50 μl zero calibrator + 5.0 mg/ml Na salicylate |
| Tracer G | 51.2% | 39.5% | with 1 mg CAHSA/tube + 50 μl zero calibrator + 1 mg/ml Na salicylate + 1 mg/ml 2,4-dinitrophenol |
| Tracer H | 58.2% | 38.6% | with 1 mg CAHSA/tube + 50 μl zero calibrator + 25 mg/ml Na salicylate + 0.15 mg/ml 2,4-dinitrophenol |

In the absence of albumin or any other protein, the binding of the analog $^{125}$I-T4 tracer to antibody at both antibody titers is of equal magnitude. In the presence of albumin-2 mg albumin/tube, contributed jointly by the tracer and the zero calibrator-the analog tracer does not bind to the higher titer antibody, while binding to the lower titer antibody at only 9.4% (tracer D). In the presence of only 1 mg albumin/tube, the binding of tracers B and C to the high titer antibody is negligible-2.6% and 1.4%, respectively -whereas binding to the lower titer antibody is significant - 18.1% and 15.0%, respectively.

The following conclusions can be drawn from the results of these experiments:

1. Albumin at concentrations of 1 to 2 mg/tube substantially binds to the tracer analog in the presence of 2.0 ng IgG antibody/tube.

2. 2.0 ng IgG antibody/tube has a lower affinity than albumin for the analog tracer.

3. In the presence of albumin blocking agents, the binding of labeled T4 analog to the antibody is restored.

The same experiments were also conducted for the free T3 assays. The tabulated results support similar conclusions (Table 2).

Table 2. Free T3.

| Ab Titer: | % B/T 1:9 000 | 1:90 000 | |
|---|---|---|---|
| Tracer A | 70.6% | 46.3% | without CAHSA: no zero calibrator (system devoid of albumin) |
| Tracer B | 6.0% | 1.0% | without CAHSA: with zero calibrator (100 µl) |
| Tracer C | 6.3% | 1.2% | with 1.0 mg/ml CAHSA tube: no zero calibrator |
| Tracer D | 5.4% | 0.9% | with 1.0 mg/ml CAHSA tube + 100 µl zero calibrator |
| Tracer E | 42.9% | 22.5% | with 1.0 mg/ml CAHSA tube + 100 µl zero calibrator + 1.0 mg/ml Na salicylate |
| Tracer F | 59.2% | 35.0% | with 1.0 mg/ml CAHSA tube + 100 µl zero calibrator + 5 mg/ml Na salicylate |
| Tracer G | 46.0% | 23.1% | with 1.0 mg/ml CAHSA tube + 100 µl zero calibrator + 1.0 mg/ml Na salicylate - 1.0 mg/ml 2.4-dinitrophenol |
| Tracer H | 37.7% | 25.5% | with 1.0 mg/ml CAHSA tube + 100 µl zero calibrator + 25 mg/ml Na salicylate - 0.15 mg/ml 2.4-dinitrophenol |

Thus, the concentration of the antibody used in a free hormone assay is critical, and must be carefully adjusted so as not to displace bound analyte from endogenous proteins. The teachings of the Amersham and Corning patents do not disclose the concentrations of the antibodies used to measure free T3 and free T4. However, based on the experiments summarized above, it can be assumed that both the Amersham and the Corning patents must have used substantially higher antibody concentrations in order to bring about reasonable binding between the antibody and the analog tracer, since neither patent employs blocking agents.

Example 2:

The working antibody concentrations established on the basis of Example 1 above are 5.5 and 2.0 ng IgG/tube of T3 antibody and T4 antibody, respectively. In order to determine the appropriate albumin blocking agent or agents for use in the free T3 and free T4 assay systems, the following compounds were added to the analog tracers in the concentrations specified. (Each tracer also contained 1 mg/ml of charcoal absorbed human serum albumin.) Maximum binding was determined for each tracer. The zero calibrator was also added to each set of maximum binding tubes.

The examples used in Tables 3 through 11 are presented here to show that, at the antibody concentrations selected, binding of the analog tracers will increase with increasing amounts of albumin blocking reagents added, until it reaches a plateau. This also shows that binding of the T3 and T4 labeled analog is eliminated by the use of an appropriate concentration of specific albumin blocking agents.

Table 3. Free T3.

| 2.4-dinitrophenol | % B/T |
|---|---|
| 10 µg/ml | 1.0% |
| 50 | 1.4% |
| 100 | 2.6% |
| 150 | 4.3% |
| 200 | 5.3% |
| 400 | 10.0% |
| 800 | 16.3% |
| 1000 | 17.2% |
| 3000 | 25.2% |
| 3500 | 27.5% |
| 4000 | 27.3% |

Table 4. Free T3.

| oleic acid | % B/T |
|---|---|
| 0.0125 mmol.l | 1.2% |
| 0.025 | 1.3% |
| 0.05 | 1.3% |
| 0.125 | 1.7% |
| 0.25 | 3.5% |
| 0.375 | 12.5% |
| 0.50 | 17.6% |
| 0.75 | 16.0% |
| 1.0 | 15.3% |

Table 5. Free T3.

| sodium salicylate | % B/T |
|---|---|
| 0.25 mg/ml | 8.8% |
| 0.50 | 14.0% |
| 1.0 | 21.3% |
| 2.0 | 26.7% |
| 5.0 | 35.8% |
| 10.0 | 36.7% |
| 20.0 | 33.3% |
| 25.0 | 30.4% |
| 30.0 | 27.8% |

Table 6. Free T3.

| sodium salicylate | 2.4-dinitrophenol | % B.T |
|---|---|---|
| 1.0 mg/ml | 1.0 mg/ml | 20.3% |
| 5.0 | 1.0 | 29.8% |
| 5.0 | 8.0 | 31.6% |
| 5.0 | 4.0 | 34.2% |
| 5.0 | 0.15 | 33.2% |
| 10.0 | 0.15 | 35.0% |
| 25.0 | 0.15 | 26.8% |

Table 7. Free T4.

| 2.4-dinitrophenol | % B/T |
|---|---|
| 10 µg/ml | 2.4% |
| 50 | 4.3% |
| 100 | 8.0% |
| 150 | 11.0% |
| 200 | 13.3% |
| 400 | 22.9% |
| 800 | 31.8% |
| 1000 | 34.1% |
| 1500 | 41.4% |
| 2000 | 45.2% |
| 2500 | 43.3% |

Table 8. Free T4.

| oleic acid | % B/T |
|---|---|
| 0.00625 mmol/l | 1.7% |
| 0.0125 | 1.7% |
| 0.025 | 1.9% |
| 0.0625 | 2.3% |
| 0.0125 | 3 5% |
| 0.1875 | 6.8% |
| 0.25 | 14.3% |
| 0.375 | 30.5% |
| 0.50 | 32.7% |
| 0.75 | 32.9% |
| 1.00 | 31.0% |

Table 9. Free T4.

| sodium salicylate | % B/T |
|---|---|
| 0.05 mg/ml | 5.3% |
| 0.075 | 7.2% |
| 0.10 | 8.5% |
| 0.15 | 11.7% |
| 0.25 | 15.6% |
| 0.50 | 22.0% |
| 1.0 | 28.3% |
| 2.0 | 37.3% |
| 5.0 | 46.0% |
| 10.0 | 45.4% |
| 20.0 | 45.0% |
| 25.0 | 40.0% |
| 30.0 | 40.0% |

Table 10. Free T4.

| sodium salicylate | 2 4-dinitrophenol | % B/T |
|---|---|---|
| 1.0 mg/ml | 1.0 mg/ml | 44.1% |
| 5.0 | 1.0 | 49.8% |
| 5.0 | 0.8 | 48.1% |
| 5.0 | 0.4 | 48.2% |
| 5.0 | 0.15 | 46.4% |
| 10.0 | 0.15 | 45.8% |
| 25.0 | 0.15 | 42.6% |

Example 3:

The following experiment was designed to demonstrate that albumin has no effect on the free T3 and free T4 assay systems.

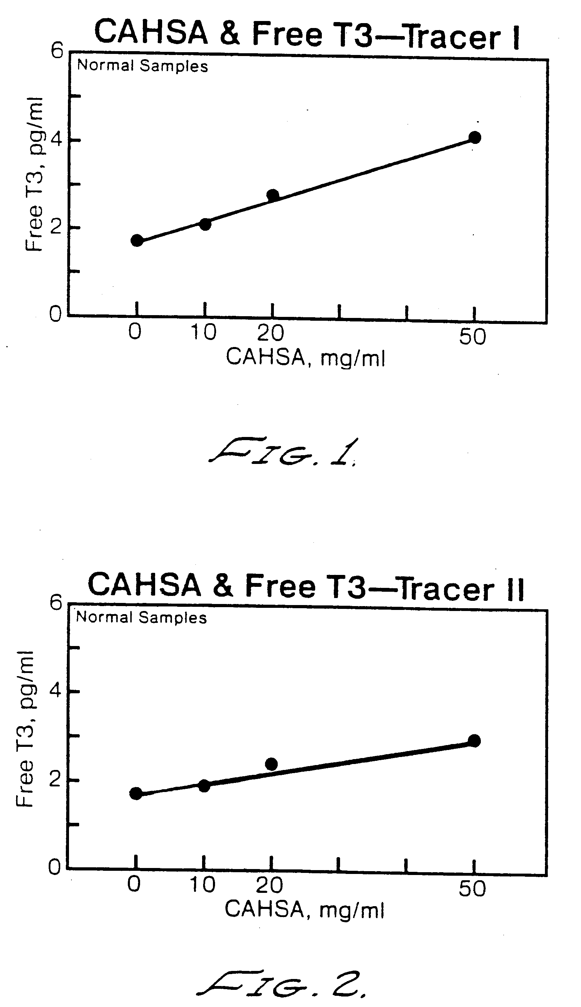

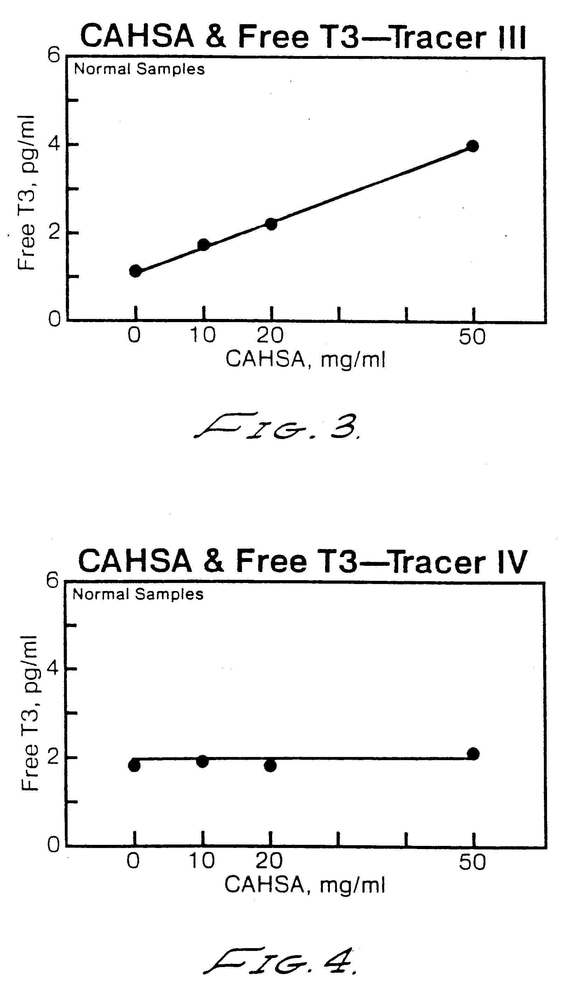

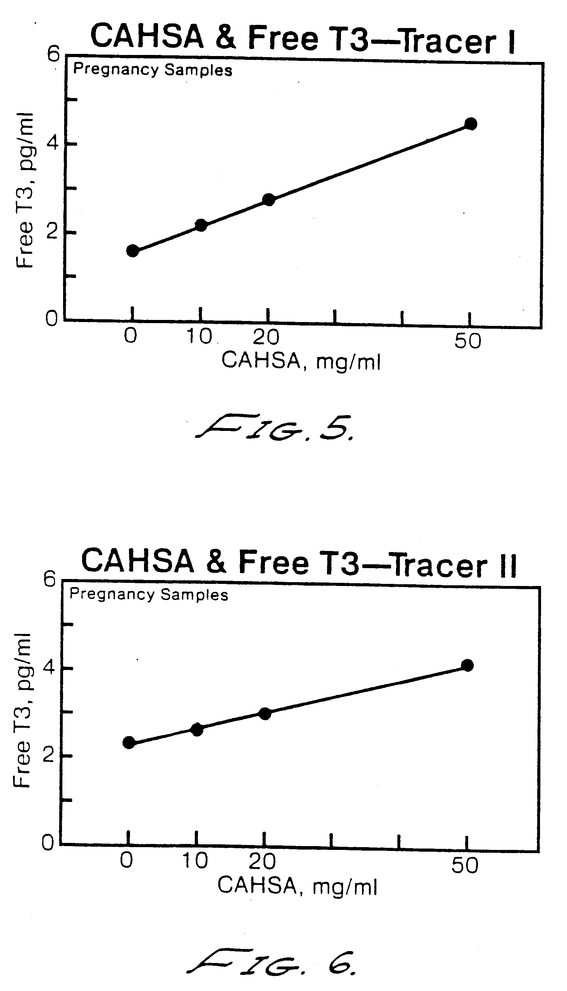

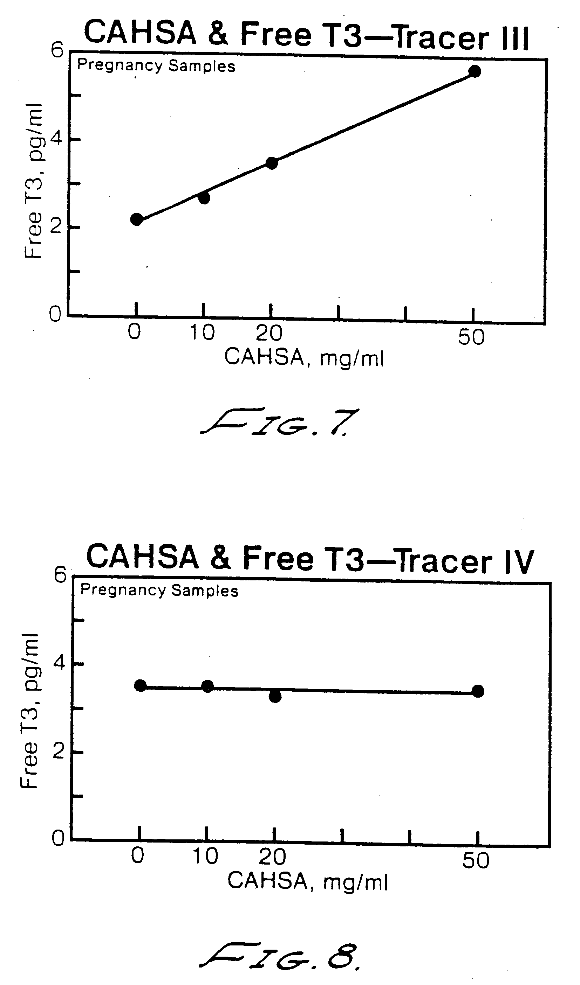

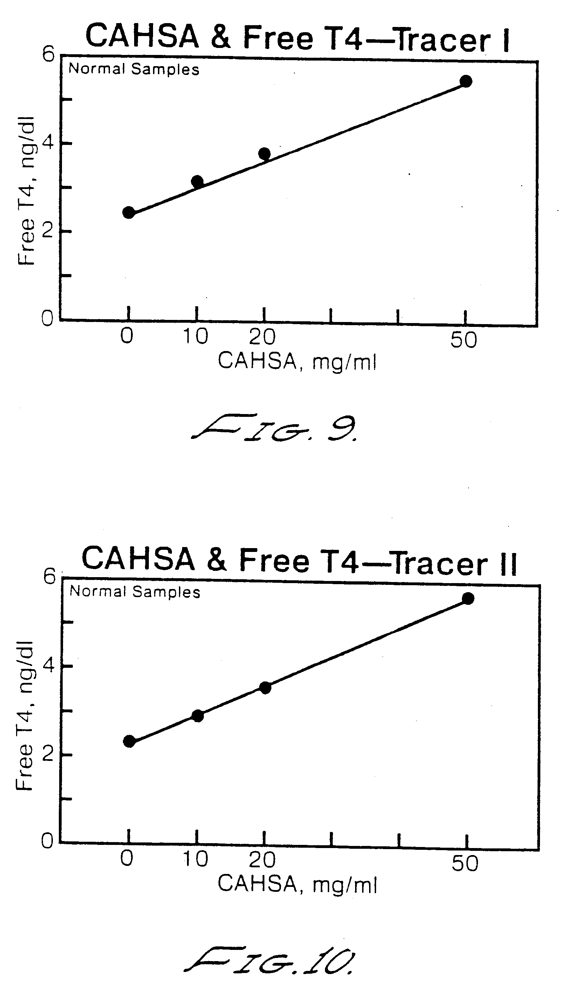

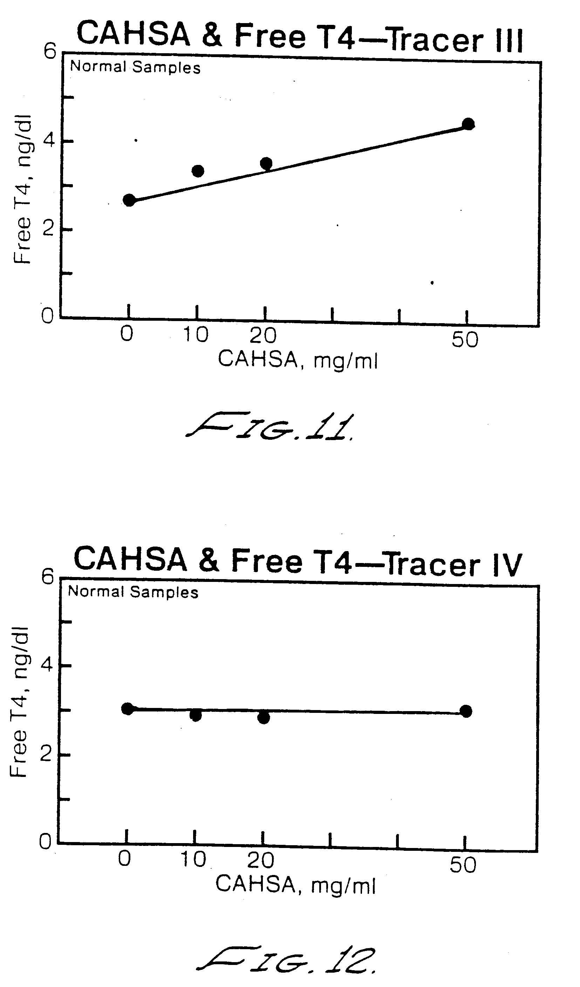

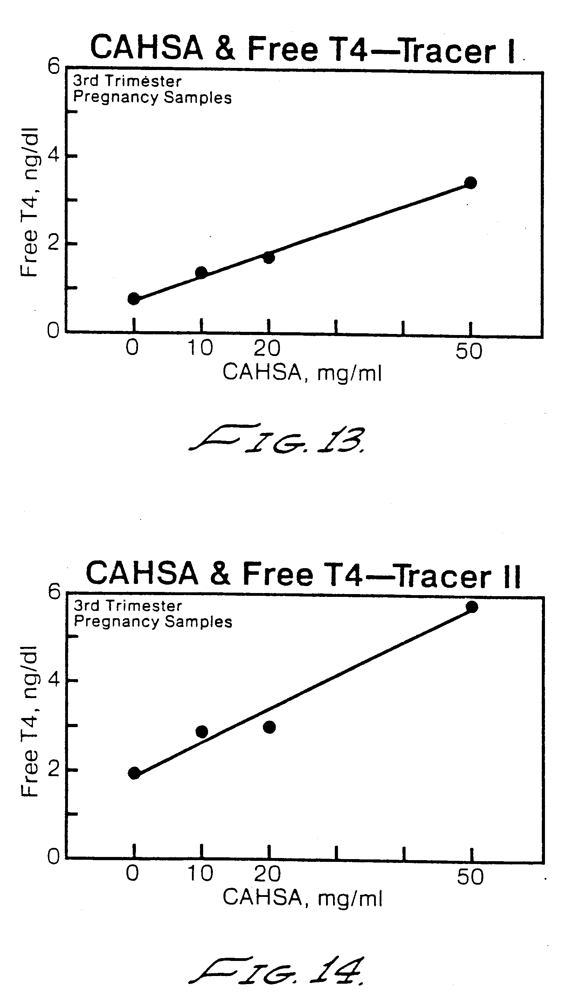

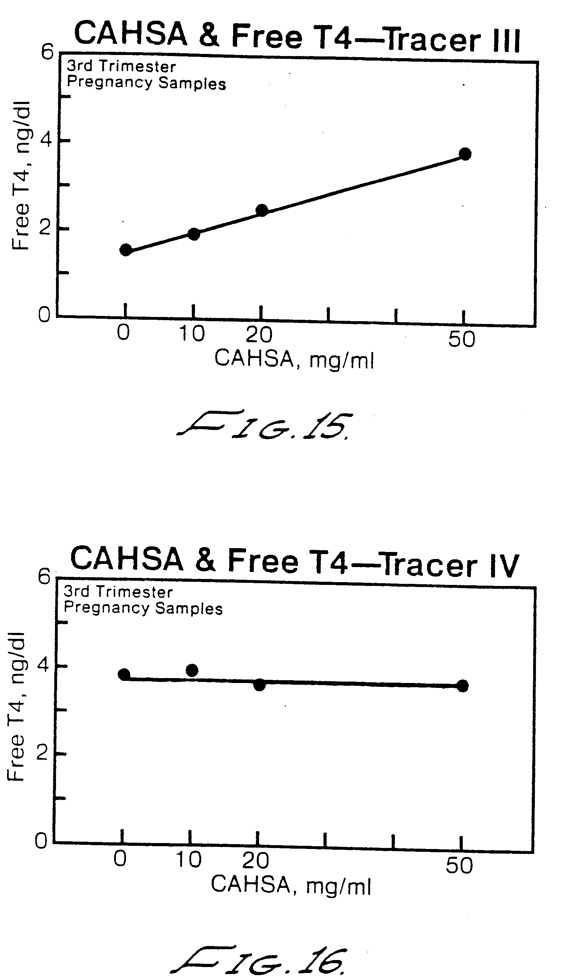

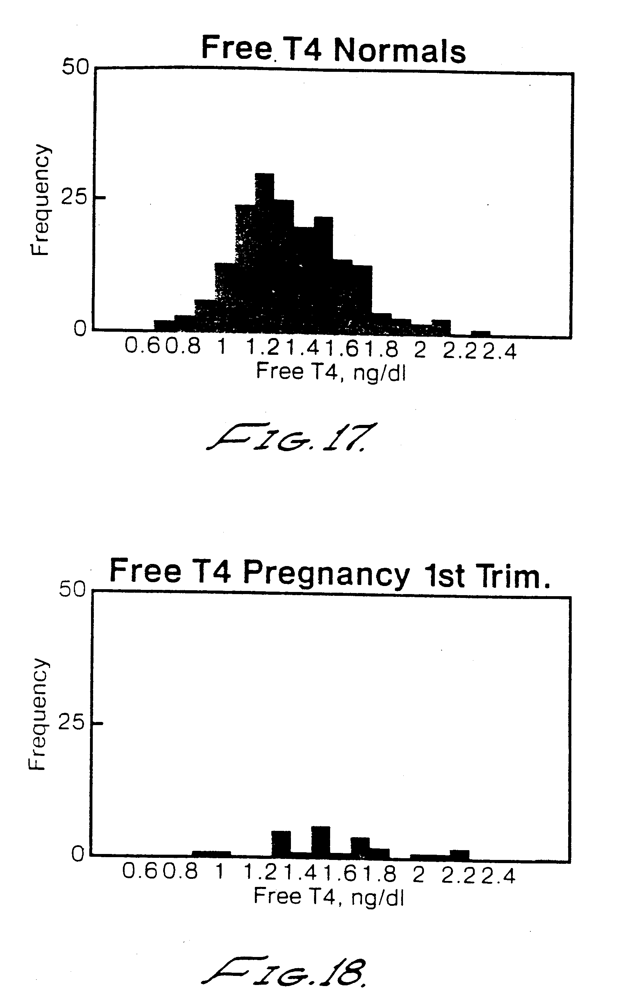

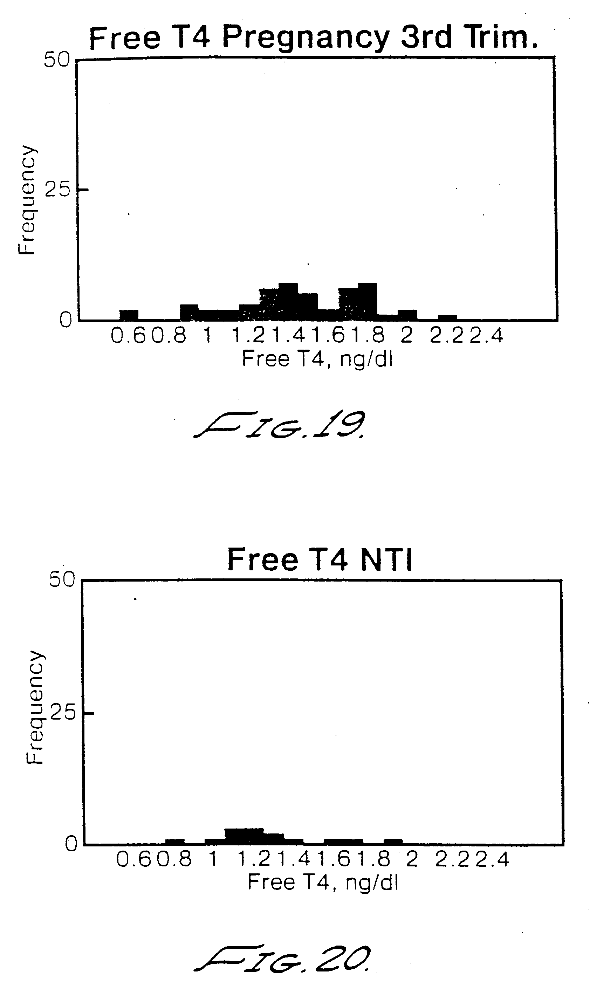

Ten samples - 5 from normal individuals and 5 from females in the third trimester of pregnancy - were each divided into 4 aliquots. To three of these aliquots, lyophilized charcoal-absorbed human serum albumin was added in concentrations of 10, 20 and 50 mg/ml. The four aliquots were then processed in duplicate, as described above, in free T3 and free T4 assays using four different tracers. The mean value for each albumin concentration (N = 5) was then plotted for each tracer (Figures 1 to 16). For free T3 and free T4, the tracers are as follows:

Table 11. Free T4 Tracers.

| | |
|---|---|
| Tracer I | contains 0.5 mg/ml sodium salicylate |
| Tracer II | contains 1 mg/ml sodium salicylate and 1 mg/ml 2,4-dinitrophenol |
| Tracer III | contains 5 mg/ml sodium salicylate |
| Tracer IV | contains 25 mg/ml sodium salicylate and 0.15 mg/ml 2,4-dinitrophenol |

Table 12. Free T3 Tracers.

| | |
|---|---|
| Tracer I | contains 1 mg/ml sodium salicylate |
| Tracer II | contains 1 mg/ml sodium salicylate and 1 mg/ml 2,4-dinitrophenol |
| Tracer III | contains 5 mg/ml sodium salicylate |
| Tracer IV | contains 25 mg/ml sodium salicylate and 0.15 mg/ml 2,4-dinitrophenol |

It is evident from the outcomes of these experiments that results generated by tracer IV for both free T3 and free T4 are unaffected by the addition of albumin up to 5.0 gm/dl, for an approximate total albumin concentration of 8.0 gm/dl.

Example 4:

In order to determine whether thyroid binding globulin (TBG) will bind the labeled free T3 and free T4 analog tracers, the following experiment was conducted using tracer IV from Example 3. TBG resin stripped of all apparent T4 and T3 was added to the respective zero calibrator for each free T4 and free T3 assay in the concentrations specified below. The observed percent bound ($B/B_o$) values are shown in the Table.

Table 13.

| | % B/$B_o$ | |
|---|---|---|
| | FT4 | FT3 |
| zero cal | 100% | 100% |
| + 10 mg/ml | 95% | 99% |
| + 20 mg/ml | 96% | 99% |
| + 50 mg/ml | 94% | 95% |

Example 4a:

This experiment was designed to check the effect of adding albumin to the zero calibrator using tracer IV from Example 3. Human serum albumin was charcoal-absorbed to remove any apparent T3 and T4 and was added to the respective zero calibrator for each free T3 and T4 in the concentrations indicated. Again, percent bound values were checked.

Table 14.

| | % B/$B_o$ | |
|---|---|---|
| | FT4 | FT3 |
| zero cal | 100% | 100% |
| + 10 mg/ml | 97% | 97% |
| + 20 mg/ml | 98% | 100% |
| + 50 mg/ml | 95% | 95% |

It is obvious from Examples 4 and 4a that neither albumin nor TBG binds the analog tracers under the conditions specified.

Example 5:

At high concentrations, sulfobromophthalein - a dye capable of binding to albumin - is able to displace T3 and T4 from the albumin molecule. Sulfobromophthalein at low concentrations is ineffective in blocking T3 and T4 analog tracers from binding to albumin. Iodinated T4 analog was compounded as described above and divided into five aliquots. To each aliquot the following reagents were added.

Table 15.

| Tracer 1 | 25 mg/ml sodium salicylate + 0.15 mg/ml 2,4-dinitrophenol (w/v) |
| Tracer 2 | 0.25 mg/ml sulfobromophthalein |
| Tracer 3 | 0.5 mg/ml sulfobromophthalein |
| Tracer 4 | 1.0 mg/ml sulfobromophthalein |
| Tracer 5 | 1.0 mmol/l oleic acid |

Each tracer was used in a separate assay for the measurement of free T4 in 20 samples under identical experimental conditions.

Considering tracer 1 as the reference and comparing the others to it, the following results were obtained.

Table 16.

|  | Tracer 1 | Tracer 2 | Tracer 3 | Tracer 4 | Tracer 5 |
|---|---|---|---|---|---|
| Total CPM | 56,145 | 59,182 | 58,591 | 55,886 | 60,030 |
| % NSB | 0.5% | 0.6% | 0.6% | 0.7% | 0.5% |
| % MB | 38.6% | 18.7% | 28.6% | 27.5% | 30.4% |
| rho * | −0.9976 | −0.9964 | −0.9973 | −0.9975 | −0.9936 |
| Calibration Range (B. B₀) 0.1 - 9.0 ng/dl | 65.3 - 8.8% | 63.4 - 7.4% | 65.1 - 9.6% | 66.6 - 10.1% | 62.8 - 3.6% |
| Intercepts. ng/dl 20% | 2.0 | 1.3 | 2.1 | 2.4 | 1.3 |
| 50% | 0.3 | 0.2 | 0.3 | 0.3 | 0.2 |
| 80% | 0.04 | 0.04 | 0.03 | 0.04 | 0.04 |
| Mean 20 samples. ng/dl | 1.3 | 0.6 | 1.2 | 1.7 | 0.7 |

* Correlation coefficient (an index of linearity)

Table 17. Regressions

| Tracer 2 = | − 0.42 Tracer 1 + 1.17 | r = | − 0.4914 |
| Tracer 3 = | 0.81 Tracer 1 + 0.14 | r = | 0.945 |
| Tracer 4 = | 1.52 Tracer 1 − 0.36 | r = | 0.956 |
| Tracer 5 = | 0.11 Tracer 1 + 0.51 | r = | 0.268 |

The results of using tracer 3 with 0.05% sulfobromophthalein correlate significantly with those obtained using tracer 1. Results generated using tracer 3 are, however, approximately 20% lower than those generated using tracer 1. Although tracer 4 correlates well with tracer 1, it yields significantly higher free T4 values, presumably due to the release of albumin bound T4 by the high concentration (0.1%) of sulfobromophthalein.

Oleic acid added to tracer 5 is capable of displacing the analog tracer from albumin. However, patient data generated with this tracer show poor correlation with data generated with tracer 1, given oleic acid at this concentration. Higher concentrations of oleic acid in the tracer-concentrations greater than 1.0 mmol/1-displace bound unlabeled T4 from albumin.

Example 6:

To examine the effects of nonesterified free fatty acids on the free T4 and free T3 assay systems, patient samples were aliquoted, lyophilized, and then reconstituted with different concentrations of oleic acid in distilled water. The reconstituted samples were assayed for free T3 and free T4 according to the protocol given above, using the same four tracers described in Example 3. The results, summarized in Table 18, indicate clearly that tracer 1 for free T4 is substantially bound to albumin, and that the addition of oleic acid displaces the tracer from albumin, producing spuriously low free T4 results. Tracers II and III are also bound to albumin, but to a much lesser degree. Tracer IV, however, is essentially unaffected by albumin, as shown in Example 3; moreover, oleic acid has no significant effect on free T4 values.

Results for free T3 are similar to those for free T4 in that they show tracer IV to be essentially unaffected by nonesterified free fatty acids, again confirming the results obtained in Example 3.

### Table 18. Effect of Oleic Acid

|  | Free T4 Tracer | | | | Free T3 Tracer | | | |
|---|---|---|---|---|---|---|---|---|
|  | I | II | III | IV | I | II | III | IV |
| Neat | 1.1 | 1.0 | 1.7 | 1.4 | 5.9 | 5.6 | 4.5 | 5.3 |
| + 2.5 mmol/1 | 0.4 | 0.9 | 1.1 | 1.3 | 2.4 | 3.2 | 3.7 | 5.3 |
| + 5.0 | 0.3 | 0.9 | 1.1 | 1.3 | 2.7 | 2.9 | 4.2 | 5.3 |
| + 7.5 | 0.3 | 0.8 | 1.1 | 1.3 | 3.2 | 2.8 | 4.2 | 5.5 |
| + 10.0 | 0.4 | 0.7 | 1.2 | 1.2 | 3.6 | 3.0 | 4.4 | 5.1 |
|  | n=3 | n=3 | n=4 | n=4 | n=3 | n=3 | n=4 | n=4 |

Example 7:

In order to establish that the results generated by the free T4 assay described above are unaffected by pregnancy and in non-thyroidal illness, 185 euthyroid samples were assayed using the tracer IV described in Example 3 above and compared to 25 first-trimester and 49 third-trimester pregnancy samples, and 14 samples from non-thyroidal illness patients. The results, summarized in Table 19 and Figures 17-20, show that there are no statistical or clinically significant differences in free T4 values during pregnancy or non-thyroidal illness as compared to a euthyroid population.

This again confirms the fact that when using appropriate albumin blocking reagents the free T4 assay is unaltered by in vivo changes in albumin concentrations.

### Table 19. Free T4.

|  | 95% | Median | N |
|---|---|---|---|
| Euthyroids | 0.8-2.0 | 1.3 | 185 |
| Pregnancy | | | |
| 1st trimester | 0.9-2.2 | 1.5 | 25 |
| 3rd trimester | 0.7-2.1 | 1.5 | 49 |
| NTI | 0.8-1.9* | 1.2 | 14 |

\* Absolute range

## Claims

1. A method for measuring the concentration of free thyroxine or triiodothyronine ligand in a biological fluid in the presence of bound ligand and endogenous binding proteins including albumin, without disturbing the equilibrium between free ligand and protein-bound ligand, which method comprises

(a) incubating a sample of the biological fluid with (i) a ligand analog tracer which, due to its chemical structure, does not bind to some of the endogenous binding proteins but does bind to at least one other endogenous binding protein including albumin, (ii) a concentration of a specific ligand binder having an affinity constant and selectivity for the free ligand such that the equilibnum between free ligand and protein-bound ligand is not disturbed and (iii) 25 mg/ml sodium salicylate and 0.15 mg/ml 2,4-dinitrophenol;

(b) separating the ligand analog tracer bound to the specific ligand binder from unbound tracer; and

(c) determining the concentration of free ligand in said biological fluid.

2. A method according to claim 1 wherein, in step (c), the concentration of free ligand in said biological fluid is determined by comparing the bound fraction of ligand analog tracer in said sample to the bound fraction in a given set of free ligand calibrators.

3. A method according to claim 1 or 2 wherein the specific ligand binder is an antibody to said free ligand.

4. A method according to any one of the preceding claims wherein the specific ligand binder is immobilized on a solid substrate.

5. A method according to claim 4 wherein the solid substrate is polypropylene.

6. A method according to any one of the preceding claims wherein the ligand analog tracer is labelled with at least one radioactive atom, an enzyme, fluorophor, light chromophore or chemiluminescent group.

7. A method according to claim 6 wherein the ligand analog tracer is N-$^{125}$I-L-triiodothyronine succinimide or N-$^{125}$I-L-thyroxine succinimide.

8. A method according to any one of the preceding claims when carried out at about 37°C and at about pH 7.4.

9. A method according to claim 2 wherein said free ligand calibrators have been prepared by adding different amounts of the ligand to ligand-free human serum, calibrating by equilibrium dialysis and assigning free ligand values.

## Patentansprüche

1. Verfahren zur Bestimmung der Konzentration von freien Thyroxin- oder Triiodthyronin-Liganden in einer biologischen Flüssigkeit in Gegenwart von gebundenem Liganden und endogenen Bindungsproteinen, einschließlich Albumin, ohne Beeinträchtigung des Gleichgewichtes zwischen freiem Liganden und Protein-gebundenem Liganden, wobei das Verfahren folgendes umfaßt:

(a) das Inkubieren einer Probe der biologischen Flüssigkeit mit (i) einem Ligandenanalogon-Tracer, welcher aufgrund seiner chemischen Struktur an einigen der endogenen Bindungsproteine nicht bindet, jedoch an mindestens einem anderen endogenen Bindungsprotein, einschließlich Albumin, bindet, (ii) einer Konzentration eines spezifischen Liganden-Bindungsstoffes mit einer Affinitätskonstanten und Selektivität für den freien Liganden, so daß das Gleichgewicht zwischen freiem Liganden und Protein-gebundenem Liganden nicht beeinträchtigt wird, und (iii) 25 mg/ml Natriumsalicylat und 0,15 mg/ml 2,4-Dinitrophenol;

(b) das Abtrennen des an dem spezifischen Liganden-Bindungsstoff gebundenen Ligandenanalogon-Tracers vom ungebundenen Tracer; und

(c) das Bestimmen der Konzentration des freien Liganden in der biologischen Flüssigkeit.

2. Verfahren nach Anspruch 1, worin in Schritt (c) die Konzentration des freien Liganden in der biologischen Flüssigkeit bestimmt wird, indem die gebundene Fraktion des Ligandenanalogon-Tracers in der Probe mit der gebundenen Fraktion in einem gegebenen Satz von Kalibratoren freier Liganden verglichen wird.

3. Verfahren nach Anspruch 1 oder 2, worin der spezifische Liganden-Bindungsstoff ein Antikörper gegen den freien Liganden ist.

4. Verfahren nach mindestens einem der vorhergehenden Ansprüche, worin der spezifische Liganden-Bindungsstoff auf einem festen Substrat immobilisiert ist.

5. Verfahren nach Anspruch 4, worin das feste Substrat Polypropylen ist.

**6.** Verfahren nach mindestens einem der vorhergehenden Ansprüche, worin der Ligandenanalogon-Tracer mit mindestens einem radioaktiven Atom, einem Enzym, Fluorophor, Lichtchromophor oder einer Chemilumineszenz-Gruppe markiert ist.

**7.** Verfahren nach Anspruch 6, worin der Ligandenanalogon-Tracer N-$^{125}$I-L-Triiodthyroninsuccinimid oder N-$^{125}$I-L-Thyroxinsuccinimid ist.

**8.** Verfahren nach mindestens einem der vorhergehenden Ansprüche, welches bei etwa 37°C und bei einem pH-Wert von etwa 7,4 durchgeführt wir.

**9.** Verfahren nach Anspruch 2, wobei die Kalibratoren freier Liganden durch Hinzusetzen unterschiedlicher Mengen des Liganden zum ligandenfreien menschlichen Serum, Kalibrieren durch Gleichgewichtsdialyse und Zuweisung von Werten für freien Liganden erhalten worden sind.

## Revendications

**1.** Méthode pour mesurer la concentration de ligand libre de thyroxine ou de triiodothyronine dans un fluide biologique en présence de ligand lié et de protéines de liaison endogènes y compris l'albumine, sans perturber l'équilibre entre ligand libre et ligand lié à une protéine, méthode qui comprend les étapes consistant à

(a) faire incuber un échantillon du fluide biologique avec (i) un traceur analogue de ligand, qui, en raison de sa structure chimique, ne se lie pas à certaines des protéines de liaison endogènes, mais qui se lie à au moins une autre protéine de liaison endogène y compris l'albumine, (ii) une concentration d'un agent de liaison de ligand spécifique ayant une constante d'affinité et une sélectivité pour le ligand libre telle que l'équilibre entre ligand libre et ligand lié à une protéine ne soit pas perturbé et (iii) 25 mg/ml de salicylate de sodium et 0,15 mg/ml de 2,4-dinitrophénol;

(b) séparer le traceur analogue de ligand lié à l'agent de liaison de ligand spécifique du traceur non lié; et

(c) déterminer la concentration de ligand libre dans ledit fluide biologique.

**2.** Méthode selon la revendication 1 dans laquelle, à l'étape (c), on détermine la concentration de ligand libre dans ledit fluide biologique en comparant la fraction liée de traceur analogue de ligand dans ledit échantillon à la fraction liée dans un ensemble donné d'étalons de ligand libre.

**3.** Méthode selon la revendication 1 ou 2 dans laquelle l'agent de liaison de ligand spécifique est un anticorps dirigé contre ledit ligand libre.

**4.** Méthode selon l'une quelconque des revendications précédentes dans laquelle l'agent de liaison de ligand spécifique est immobilisé sur un substrat solide.

**5.** Méthode selon la revendication 4 dans laquelle le substrat solide est du polypropylène.

**6.** Méthode selon l'une quelconque des revendications précédentes dans laquelle le traceur analogue de ligand est marqué avec au moins un atome radioactif, une enzyme, un fluorophore, un chromophore sensible à la lumière ou un groupe chimioluminescent.

**7.** Méthode selon la revendication 6 dans laquelle le traceur analogue de ligand est le N-$^{125}$I-L-triiodothyronine succinimide ou le N-$^{125}$I-L-thyroxine succinimide.

**8.** Méthode selon l'une quelconque des revendications précédentes effectuée à environ 37°C et à environ pH 7,4.

**9.** Méthode selon la revendication 2 dans laquelle lesdits étalons de ligand libre ont été préparés en différentes quantités du ligand à du sérum humain sans ligand, en étalonnant par dialyse à l'équilibre et en assignant des valeurs de ligand libre.

## CAHSA & Free T3—Tracer I

Normal Samples

Free T3, pg/ml

CAHSA, mg/ml

*FIG. 1.*

## CAHSA & Free T3—Tracer II

Normal Samples

Free T3, pg/ml

CAHSA, mg/ml

*FIG. 2.*

14

## CAHSA & Free T3—Tracer III

*FIG. 3.*

## CAHSA & Free T3—Tracer IV

*FIG. 4.*

FIG. 5.

FIG. 6.

FIG. 7

FIG. 8.

17

## CAHSA & Free T4—Tracer I

*FIG. 9.*

## CAHSA & Free T4—Tracer II

*FIG.10.*

# CAHSA & Free T4—Tracer III

*FIG.11.*

# CAHSA & Free T4—Tracer IV

*FIG.12.*

## CAHSA & Free T4—Tracer I

3rd Trimester
Pregnancy Samples

*FIG. 13.*

## CAHSA & Free T4—Tracer II

3rd Trimester
Pregnancy Samples

*FIG. 14.*

# CAHSA & Free T4—Tracer III

*FIG. 15.*

# CAHSA & Free T4—Tracer IV

*FIG. 16.*

## Free T4 Normals

*FIG.17.*

## Free T4 Pregnancy 1st Trim.

*FIG.18.*

## Free T4 Pregnancy 3rd Trim.

*FIG.19.*

## Free T4 NTI

*FIG.20.*